# EUROPEAN PATENT SPECIFICATION

(11) **EP 4 347 865 B1**
(45) Date of publication and mention of the grant of the patent: **07.05.2025**
(21) Application number: 22729314.9
(22) Date of filing: 19.05.2022
(51) Int. Cl.: C12Q 1/6804

(54) **METHOD FOR SENSITIVE ANALYTE DETECTION ASSAYS AND KITS THEREFOR**
VERFAHREN FÜR SENSITIVE ANALYTNACHWEISTESTS UND KITS DAFÜR
PROCÉDÉ POUR DOSAGES DE DÉTECTION D'ANALYTES SENSIBLES ET KITS ASSOCIÉS

(30) Priority: 25.05.2021 SE 2150659
(43) Date of publication of application: 10.04.2024
(73) Proprietor: Cavidi AB, 754 50 Uppsala (SE)
(72) Inventor: KÄLLANDER, Clas, 760 49 Herräng (SE); PETTERSSON, Ingvar, 754 27 Uppsala (SE); FRIDBORG, Ingela, 756 51 Uppsala (SE)
(74) Representative: Zacco Sweden AB
(86) International application number: PCT/SE2022/050491
(87) International publication number: WO 2022/250596

(56) References cited:
- WO-A2-2005/081908
- VAN DESSEL ET AL: "Assessment of the diagnostic potential of immuno-RCA in 96-well ELISA plates for foot-and-mouth disease virus", JOURNAL OF VIROLOGICAL METHODS, ELSEVIER BV, NL, vol. 147, no. 1, 25 October 2007 (2007-10-25), pages 151 - 156, XP022390482, ISSN: 0166-0934
- ZHOU Y ET AL: "IN SITU DETECTION OF MESSENGER RNA USING DIGOXIGENIN-LABELED OLIGONUCLEOTIDES AND ROLLING CIRCLE AMPLIFICATION", EXPERIMENTAL AND MOLECULAR PATHOLOGY, ACADEMIC PRESS, US, vol. 70, no. 3, 1 June 2001 (2001-06-01), pages 281 - 288, XP001133841, ISSN: 0014-4800, DOI: 10.1006/EXMP.2001.2365
- NONG RACHEL YUAN ET AL: "DNA-assisted protein detection technologies", EXPERT REVIEW OF PROTEOMICS, FUTURE DRUGS, LONDON, GB, vol. 9, no. 1, 1 January 2012 (2012-01-01), pages 21 - 32, XP009186898, ISSN: 1744-8387, DOI: 10.1586/EPR.11.78

## Description

### Field of the invention

This invention relates generally to the fields of detection of biomolecules. More specifically, it relates to the use of nucleic acid polymerization to amplify the detectable signal in ligand-analyte binding assays.

### Introduction

Ligand binding assays represent powerful tools to identify and quantitate a wide range of compounds, such as drugs, antigens and antibodies. One class of ligand binding assays use antibodies as the ligand binding component. Enzyme immunoassays exist in a variety of formats, such as ELISA (enzyme linked immunosorbent assay), and CLIA, (chemiluminescence immunoassay). The classical enzyme immunoassays achieves in best case the one pg/ml sensitivity. For a typical protein with the molecular weight (Mw) approximately equal to 20 kDaltons, one pg/ml is equivalent to approximately 3×10⁷ molecules/ml or 50 attomoles/ml. This sensitivity has been considered sufficient for almost all biomedical tasks.

In recent years, however, many new challenges in biomedical diagnostics suggest that a few hundred-fold increase of sensitivity is required for optimal detection of low-abundance biomarkers. Another obvious advantage of increased sensitivity is that interfering effects of the biological matrix can be counteracted by a simple dilution of samples without loss in assay performance.

In the classical enzyme immunoassays, detection is not possible below a certain number of molecules, due to limitations in the signal generating systems and due to the system background, i.e. unspecific binding of the signal generating component to material in the system.

Several techniques with improved sensitivity; such as DNA-enhanced immunoassays (immuno-Polymerase Chain Reaction (iPCR)), (Cantor et al, Science, Vol. 258, 2 Oct. 1992, US 5665539), electrochemiluminescence (Wei and Wang 2011) and single molecule counting (Todd et al 2007) have emerged to meet these demands. The following text will mainly focus on immuno-PCR, that is the prior art most relevant for the current invention.

International patent publication no. WO 91/17442 describes protein/nucleic acid hybrid probes that are used to enhance the signal in immunoassays. The probe comprises a protein moiety, a double stranded polynucleotide functioning as a promoter for a DNA dependent RNA polymerase, and a single or double stranded template for the promoter. The protein moiety is a polypeptide capable of functioning as an antibody which binds specifically to a known antigen. The transcription products are quantified in a separate step by use of labeled complementary polynucleotide probes.

Cantor et al, US 5665539, Science, Vol. 258, 2 Oct. 1992, mentioned above, have reported the attachment of oligonucleotides to antibodies in order to permit detection of bound antigen. A streptavidin protein-A chimera that possesses tight and specific binding affinity for both biotin and immunoglobulin G was used to attach biotinylated DNA specifically to antigen-monoclonal antibody complexes that had been immobilized on micro titer plate wells. A segment of the attached DNA was then amplified by PCR (Polymerase Chain Reaction). Analysis of the PCR products allowed detection of down to 9.6×10⁻²² moles) which is a significant improvement compared to conventional ELISA. However, in Cantor et al., extra steps are required for addition of biotinylated reagents and binding proteins. Each assay may include as many as 20 wash steps to remove excess reagents and free the assay of non-specifically bound reagents. These reagent additions and wash steps add complexity and time to the immuno-PCR procedures.

Hendrickson et al., Nucleic Acids Research, 1995, Vol 23, No.3, report an improvement of the Cantor et al. assay that reduces complexity. Several reaction and wash steps were eliminated by labeling antibody with DNA by direct covalent linkage of the DNA to the antibody. The PCR products in these early immune PCR assays were separated on agarose gel electrophoresis and detected by various methods. The assays for detection/ quantification of both nucleic acids and protein has since then been improved by use of more efficient methods for detection of the PCR products. Modern real time PCR assays utilize fluorophore labeled nucleotides that after excitation at the required wavelength can be measured continuously after each amplification cycle (for a review see Kralik, & Ricchi 2017).

Theoretically, immuno PCR has the capacity to improve the detection sensitivity of ligand binding assays several orders of magnitude. The improvement is, however, in practice often limited to three to ten times (e.g. Simonova et al 2018). The critical limiting factor is often system backgrounds. Label systems increasing positive signals does in general also increase the signals from background controls. US 2005/0233351Al addressed these problems. Two different antibody being specific for different determinants of the antigen and conjugated with cross-linkable oligonucleotides are bound to the antigen. The oligonucleotides are amplified, whereby only such oligonucleotides will be amplified which have been cross-linked to each other. In this way, unspecific background signals are avoided or at least reduced.

The basic problems affecting all PCR tests also hampers immuno PCR. The systems are sensitive for contamination leading to false positivity. Another obstacle is to design relevant amplification controls to allow quantitative detection. Further, there are set of problems more specific for immuno PCR, such as the repeated high temperature cycling adversely affecting the antibody-antigen interaction. The structure of both the antibody and the antigen, if it is a peptide or protein, may be affected resulting in a weaker antibody-antigen binding. The gain in sensitivity from the signal amplification can thus be counteracted by the decreased strength of the antibody-antigen binding.

There are a few amplification methods that do not use temperature cycling. Immune-rolling-circle amplification (RCA) has been used for detection of both proteins and nucleic acids (Van Dessel et al, J Virol Methods. vol 147, 2008, Zhou et al, Exp Mol Pathol. Vol 70, 2001). RCA is based upon use of probes or antibodies labelled with primers. The probes/antibodies bind to their target and a reaction solution containing a circular ssDNA template, with a sequence complementary to the primer, labelled nucleosides, a polymerase such as Ø29 DNA polymerase or Bst exo-DNA polymerase and other necessary reaction components is added. Starting from the primer end a single stranded DNA of practically unlimited length can be produced. This long ssDNA is sensitive to nucleases and mechanical brakes e.g., during wash procedures. As a result, this method is relevant for qualitative detection, but less relevant for quantitative analysis.

WO2006/053380 discloses a method of screening for the presence or absence of an analyte in a sample comprising the use of e.g. antibodies specific to the analyte and conjugated to a polynucleotides, the method comprising extending the polynucleotide, or producing a complementary polynucleotide, and detecting the so extended or produced polynucleotide. The long single stranded extended polynucleotides required to achieve a significant signal amplification according to this method would be fragile. Both mechanical brakes during the washing procedures and effects of nucleases originating from the samples and/ or regents will interfere, generate irregularities, limit the signal amplification and hamper the usefulness of this method. WO2006/053380 does not elaborate on, or enable, the embodiment relating to production of a complementary polynucleotide, and provides no examples based on this embodiment. It is thus unclear if this model is practically useful and/ or provide an increase detection sensitivity compared to standard ELISA tests.

For the avoidance of doubt, any discussion above about drawbacks or shortcomings of the prior art is based on the present inventors' understanding of the technologies in view of the present invention and shall not be considered as an admission that such drawbacks and shortcomings were recognized in the art or were known, understood, or otherwise obvious to a person of ordinary skill in the art.

### Summary of the invention

The present invention aims to solve at least some of the shortcomings of the prior art. Accordingly, a simple and sensitive method for detection of biological or chemical analytes is provided that does not require temperature cycling or temperatures above 37 °C, requires few steps and reagents, and only standard equipment.

The invention in a first aspect relates to a method for detection of an analyte of interest in a sample comprising
- bringing a first ligand capable of specific binding to the analyte in contact with the sample under conditions facilitating specific binding of the ligand to the analyte, wherein the first ligand comprises a binding moiety capable of specific binding to the analyte and a primer oligo-deoxyribonucleotide bound to the binding moiety wherein the primer oligo-deoxyribonucleotide has a length of 18-40 nucleotides;
- removing unbound first ligand from the sample;
- bringing the bound first ligand in contact with a template polyribonucleotide being longer than the primer oligo-deoxyribonucleotide and having a sequence that is at least partially complementary to the primer oligo-deoxyribonucleotide; a plurality of labelled deoxyribonucleotide triphosphates; and an enzyme with reverse transcriptase activity;
- incubating the bound first ligand comprising the attached primer oligo-deoxyribonucleotide, the template polyribonucleotide, the labelled deoxyribonucleotide triphosphates, and the enzyme under conditions suitable for synthesis of a labelled DNA-strand by said enzyme; and
- detecting any synthesized labelled DNA strand.

In one embodiment, the primer oligo-deoxyribonucleotide is selected from oligo-deoxyribonucleotides containing all four bases, A,G,T and C or a subset thereof or from oligo-deoxyribonucleotides containing a single base, preferably oligo-deoxythymidine.

In one embodiment, the labelled deoxyribonucleotides are labelled deoxyuridine triphosphates (dUTP), such as bromo-dUTP or iodo-dUTP, preferably bromo-dUTP, and the template polyribonucleotide is polyriboadenylic acid.

In one embodiment, the primer oligo-deoxyribonucleotide has a length of 22-40 nucleotides.

In one embodiment, the template polyribonucleotide has a length of 10²-10³ nucleotides, preferably 2*10²-5*10² nucleotides.

In one embodiment, the enzyme with reverse transcriptase activity is a eukaryotic polymerase, such as DNA polymerase gamma; a viral polymerase, such as a retroviral reverse transcriptase, preferably HIV or M-MuLV reverse transcriptase, or a bacterial polymerase, such as a bacterial polymerase is derived from mesophilic bacteria, preferably the Klenow fragment of *E.coli* DNA polymerase I , or a bacterial polymerase derived from thermophilic bacteria, preferably Bst 3.0 derived from *Bacillus stearothermophilus.*

In one embodiment, the enzyme with reverse transcriptase activity is selected from the group consisting of DNA polymerase gamma, HIV or M-MuLV reverse transcriptase, the Klenow fragment of *E.coli* DNA polymerase I, and Bst 3.0 derived from *Bacillus Stearothermophilus.*

In one embodiment, the incubation step is performed at a constant temperature, preferably at 37 °C, for 30 min-4hrs, preferably for 1hr.

In one embodiment,the detection is performed through a detection method comprising the steps
- bringing a second ligand capable of specific binding to the labelled DNA strand in contact with the synthesized labelled DNA strand under conditions facilitating specific binding of the second ligand to the DNA strand, wherein the second ligand comprises a binding moiety capable of specific binding to the synthesized labelled DNA and a covalently attached enzyme;
- removing the unbound second ligand from the sample;
- adding a substrate for the attached enzyme that upon cleavage by the attached enzyme generates a detectable signal; and
- detecting said signal.

In one embodiment, the second ligand is an antibody which specifically binds to the synthesized labelled DNA having covalently attached thereto an enzyme, such as alkaline phosphatase or horse radish peroxidase.

In one embodiment, the covalently attached enzyme acts on a substrate to generate a colorimetric, fluorescent, chemiluminescent or electrochemical signal

In one embodiment, the covalently attached enzyme is alkaline phosphatase which acts on a substrate to generate a chemiluminescent signal as the detectable signal.

In one embodiment, the detection of the analyte of interest is a quantitative detection.

In a second aspect, the invention relates to a kit of parts comprising a first ligand comprising a binding moiety, capable of specific binding to an analyte of interest, and a primer oligo-deoxyribonucleotide of a length of 18-40 nucleotides bound to the binding moiety; a template polyribonucleotide being longer than the primer oligo-deoxyribonucleotide and having a sequence that is at least partially complementary to the primer oligo-deoxyribonucleotide, a plurality of labelled deoxyribonucleotide triphosphates; and an enzyme with reverse transcriptase activity.

In one embodiment, the primer oligo-deoxyribonucleotide. is selected from oligo-deoxyribonucleotides containing all four bases, A,G,T and C or a subset thereof or from oligo-deoxyribonucleotides containing a single base, preferably oligo-deoxythymidine.

In one embodiment, the labelled deoxyribonucleotide triphosphates are labelled deoxyuridine triphosphate (dUTP), such as bromo-dUTP or iodo-dUTP, preferably bromo-dUTP, and the template polyribonucleotide is polyriboadenylic acid.

In one embodiment, the primer oligo-deoxyribonucleotide has a length of 22-40 nucleotides.

In one embodiment, the template poly-ribonucleotide has a length of 10²-10³ nucleotides, preferably 2*10²-5*10² nucleotides.

In one embodiment, the enzyme with reverse transcriptase activity is a eukaryotic, viral or bacterial polymerase, such as DNA polymerase gamma, HIV or M-MuLV reverse transcriptase, the Klenow fragment of *E.coli* DNA polymerase I or Bst 3.0 derived from *Bacillus Stearothermophilus.*

In one embodiment, the kit of parts further comprises a second ligand capable of specific binding to the labelled deoxyribonucleotides, said second ligand also comprising a covalently attached enzyme; and a substrate for said enzyme.

In one embodiment, said second ligand is a monoclonal antibody having covalently attached thereto an enzyme, such as alkaline phosphatase or horse radish peroxidase

In one embodiment, the covalently attached enzyme is alkaline phosphatase which acts on a substrate to generate a chemiluminescent signal as the detectable signal.

### Brief description of drawings

Fig1 show a schematic description of the polymerase boosted signal amplification model according to the invention
Fig. 2 shows the relationship between the signal generated and the amount of primer labelled ligand in one embodiment of the invention, illustrating the ability of the system to detect and quantitate primers that are used as label for analyte detection.
Fig 3 illustrates the relationship between ligands with oligo deoxythymidine primers of different length and signal.
Fig. 4 illustrates use of reverse transcription by eukaryotic, retroviral and bacterial polymerases in the primer extension step.
Fig. 5 illustrates the relationship between different amounts of labelled ligand and signal.
Fig. 6 illustrate the relationship between different amounts of reverse transcriptase activity used and signal.
Fig 7 illustrates the relationship between reverse transcription reaction time for two retroviral reverse transcriptases and signal.
Fig. 8 illustrates the use of different labeled deoxyribonucleotide triphosphates in the primer extension step.
Figure 9 illustrates an implementation of the invention in an antigen capture ELISA.

### Definitions and abbreviations

"odTX" is an abbreviation for oligo deoxythymidine of length X deoxythymidine nucleotide units, i.e. "odT22" is an oligonucleotide consisting of 22 deoxythymidines.

"prA" is an abbreviation for single stranded polyriboadenylic acid.
ELISA = enzyme linked immunosorbent assay
PCR = Polymerase Chain Reaction
RT = Reverse transcriptase
SARS = severe acute respiratory syndrome
CoV = Corona Virus
NIBSC = National Institute for Biological Standards and Control (UK)
M-MuLV = Moloney Murine leukemia virus
HIV = Human Immunodeficiency virus
HRP = horseradish peroxidase
AP= alkaline phosphatase
Bst = *Bacillus Stearothermophilus*
Pol-G = human gamma polymerase

### Detailed description

The present invention relates to a method for detection, in particular quantitative detection, of an analyte of interest in a sample comprising the use of a ligand capable of specific binding to the analyte, wherein the ligand comprises a primer oligo-deoxyribonucleotide having a length of 18-40 nucleotides, a template polyribonucleotide being longer than the primer oligo-deoxyribonucleotide and having a sequence that is at least partially complementary to the primer oligo-deoxyribonucleotide, a plurality of labelled deoxyribonucleotide triphosphates, and an enzyme with reverse transcriptase activity, wherein the analyte is detected by detecting any labelled DNA strand synthesized by the enzyme. The invention also relates to a kit comprising reagents for performing such a method.

The current invention utilizes affinity ligands such as antibodies that are able to specifically bind to an analyte of interest. The affinity ligands are labeled with short single stranded deoxyoligonucleotides that serve as primers in a reverse transcription reaction constituting a primer extension step. No temperature cycling or elevated temperature is needed, only one incubation at a constant, physiological temperature is required. In the reverse transcription step, a DNA-RNA helix is formed wherein the deoxyribonucleotides incorporated into the DNA strand are labeled so as to be detectable in a subsequent detection step.

In the current invention, for each such affinity ligand bound to an analyte molecule, the labeled deoxyribonucleotides in the newly made DNA strand in the DNA-RNA hybrid will be the target molecules for a subsequent signal generation step. This means more signal-generating conjugates bound per affinity ligand/analyte complex providing a basis for the signal amplification, as compared to a standard immunoassay wherein the bound antibody is the sole target molecule for a subsequent signal generation step (e.g a second antibody-enzyme conjugate).

The current invention also reduces the complexity of the nucleic acid reagents needed in analyte detection methods, as compared to prior art immuno-PCR, by using one universal DNA primer sequence and one universal RNA template for a primer extension step. Both oligo/polynucleotides are composed of a repeated single nucleotide, such as deoxy thymidine and adenosine respectively. No temperature cycling or elevated temperature is needed, only one incubation at physiological temperature.

The enzymatic polymerization process used for primer extension is a reverse transcription reaction, whereby the polymerase enzyme builds a new complementary DNA strand. To do this it needs an RNA strand to copy (template), a short complementary DNA strand to start extending from (primer) and complementary deoxynucleotide triphosphates (dNTPs) that can be used as building blocks for the growing strand. The dNTPs used in the present invention are labeled to facilitate detection of the newly synthesized DNA strand.

One advantage of using a reverse transcription reaction instead of a DNA dependent DNA polymerase reaction like in immuno PCR is that reverse transcriptase activity is much less ubiquitous in biological samples, as compared to DNA polymerase activity. Contamination from endogenous DNA polymerases could cause increased background signals and false positive results in negative samples. Specific reverse transcriptases are of retroviral or synthetic origin and the baseline level of reverse transcriptase activity in mammalian cells is very low. Some DNA polymerases may be coaxed to perform reverse transcription in *in vitro* systems but the reverse transcriptase activity is much lower than the DNA polymerase activity.

Thus, in one aspect, the present invention relates to a method for detection of an analyte of interest in a sample comprising
- bringing a first ligand capable of specific binding to the analyte in contact with the sample under conditions facilitating specific binding of the ligand to the analyte, wherein the first ligand comprises a binding moiety capable of specific binding to the analyte and a primer oligo-deoxyribonucleotide bound to the binding moiety wherein the primer oligo-deoxyribonucleotide has a length of 18-40 nucleotides;
- removing unbound first ligand from the sample;
- bringing the bound first ligand in contact with a template polyribonucleotide being longer than the primer oligo-deoxyribonucleotide and having a sequence that is at least partially complementary to the primer oligo-deoxyribonucleotide; a plurality of labelled deoxyribonucleotide triphosphates; and an enzyme with reverse transcriptase activity;
- incubating the bound first ligand comprising the attached primer oligo-deoxyribonucleotide, the template polyribonucleotide, the labelled deoxyribonucleotide triphosphates, and the enzyme under conditions suitable for synthesis of a labelled DNA-strand by said enzyme; and
- detecting any synthesized labelled DNA strand.

In some embodiments, the primer oligo-deoxyribonucleotide is selected from oligo-deoxyribonucleotides containing all four bases, A,G,T and C or a subset thereof or from oligo-deoxyribonucleotides containing a single base, preferably oligo-deoxythymidine.

In some embodiments, the template poly-ribonucleotide has a length of 10²-10³ nucleotides, such as about 2*10²-5*10², nucleotides. In some embodiments, the current invention utilizes reverse transcriptases or DNA polymerases with reverse transcriptase activity, oligo-deoxythymidine (odT) labeled antibodies and soluble poly(rA) templates with an average length of approximately 500 Abases for primer extension. After the reverse transcription step each bound oligo-deoxythymidine (odT) labeled antibody will carry a RNA-DNA hybrid molecule where the newly made DNA will be composed of chemically labeled bases.

In some embodiments, the enzyme with reverse transcriptase activity is a eukaryotic, viral or bacterial polymerase, such as DNA polymerase gamma, a retroviral reverse transcriptase such as HIV or M-MuLV reverse transcriptase, the Klenow fragment of *E.coli* DNA polymerase I or Bst 3.0 derived from *Bacillus Stearothermophilus.* The *in vitro* processivity of the different polymerases varies dependent on the reaction conditions, but a typical value is a half-life of the enzyme primer template complex of 2-3 min.

In some embodiment, the incubation step is performed at a constant temperature, preferably at 37 °C, for 30 min-4hrs, preferably for 1hr. *In vitro* synthesis on prA templates by retroviral reverse transcriptase (RT) is more or less processive with an incorporation rate of 10-15 nucleotides/s at 37°C (see e.g. Hubert et al 1989). From these assumptions it can be calculated that enzymatic elongation of an odT primer will reach the end of a prA₅₀₀ template within 30-50 seconds. A traditional reaction model, utilizing one template only, will neither provide sufficient signal amplification for the current invention, nor a polymerization reaction linear with time. Certain polymerases have, however, the ability to use multiple templates starting from a single primer leading to products longer than template length. This type of reactions can be performed by retroviral reverse transcriptase and certain other more promiscuous polymerases and is an *in vitro* reflection of the reactions the enzymes catalyze *in vivo* (e.g. Hubert et al 1989, Lennerstrand et al 1996).

In one embodiment, the detection is performed through a detection method comprising the steps
- bringing a second ligand capable of specific binding to the labelled DNA strand in contact with the synthesized labelled DNA strand under conditions facilitating specific binding of the second ligand to the DNA strand, wherein the second ligand comprises a binding moiety capable of specific binding to the synthesized labelled DNA and a covalently attached enzyme;
- removing the unbound second ligand from the sample;
- adding a substrate for the attached enzyme that upon cleavage by the attached enzyme generates a detectable signal; and
- detecting said signal.

In some embodiments, wherein said second ligand is an antibody which specifically binds to the synthesized labelled DNA having covalently attached thereto an enzyme, such as alkaline phosphatase or horse radish peroxidase. Such covalently attached enzyme may act on a substrate to generate a colorimetric, fluorescent, chemiluminescent or electrochemical signal. In some embodiments, the enzyme is alkaline phosphatase. In some embodiments, the enzyme which acts on a substrate generates a chemiluminescent signal as the detectable signal. A number of suitable substrates are commercially available, such as AMPPD (CAS no: 122341-56-4) and ready-to-use substrate solutions under various trade names such as DynaLight^{™} from ThermoFisher Scientific.

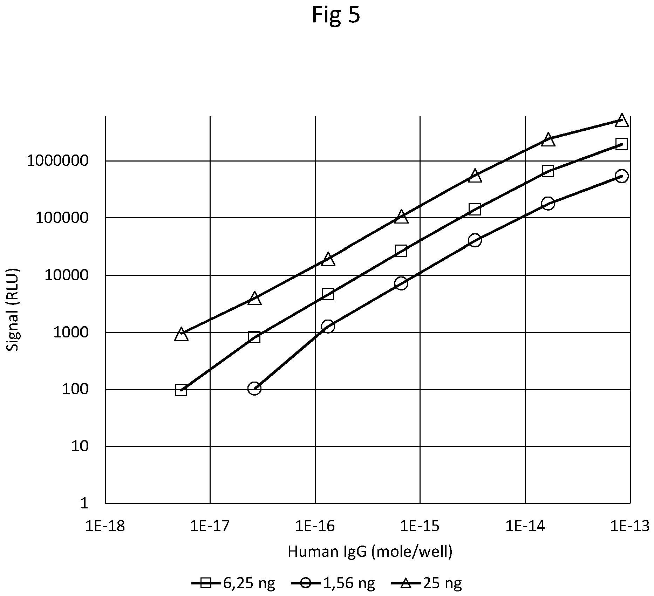

In some embodiments, the detection of the analyte of interest is a quantitative detection. In these embodiments, the amount or concentration of analyte of interest in the sample is quantitated based on the amount of detected synthesized labelled DNA strand and/or the intensity of the detected signal. As illustrated in e.g. Example 4 and Figure 5, there is a substantially linear relationship between the amount of analyte and generated signal over a wide detection range. The signal level may also be affected by other factors, such as amount of enzyme activity and polymerization reaction time. The assay method according to the present invention can thus be set up for practical use by running calibration assays under known conditions (analyte amount ranges, reagent concentrations, reaction volumes, reaction temperature, reaction time, etc.) to establish the correlation between analyte amounts/concentrations on one hand and amounts of detected synthesized labelled DNA strand and/or the intensity of the detected signal on the other, in a desired range of analyte amounts/concentrations. Such correlations are then used to quantify the amount or concentration of the analyte of interest in the sample when subjecting the sample to the methods according to the invention.

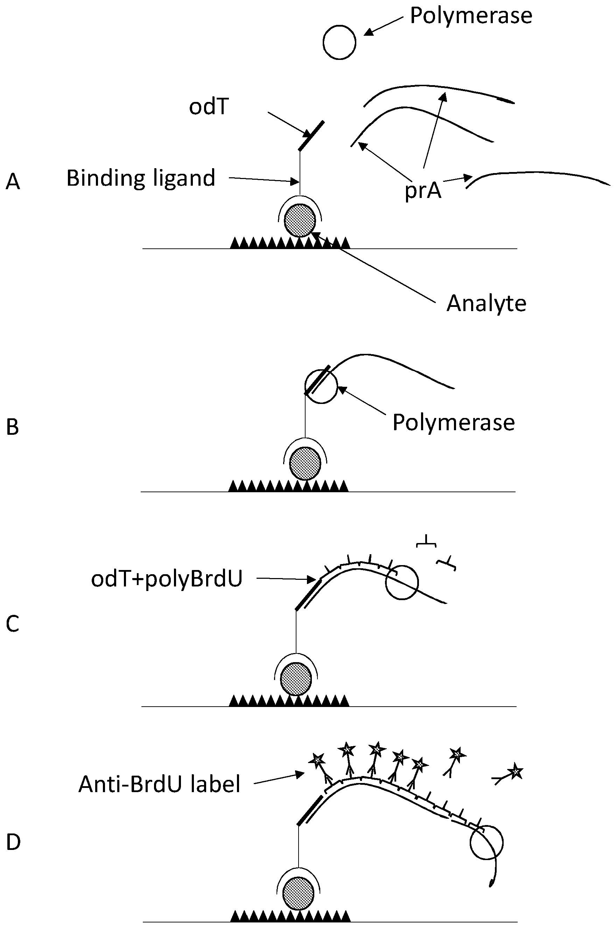

An overview of the steps of one embodiment of the method according to the invention is provided in Fig.1. A) The analyte is directly or indirectly bound to a solid phase. An affinity ligand labeled with a short primer, e.g. an antibody conjugated with odT22, is bound to the immobilized analyte. After an incubation, non bound affinity ligand is removed by washing. A reaction solution containing a template at least partially complementary to the primer, one or several modified nucleotide triphosphates and other buffer components, except the primer, needed for the reverse transcription reaction are added. The primer associates to the template by base pairing. B) An enzyme with reverse transcriptase activity is added and binds to the 3' end of the primer in the primer template complex. C) The enzymatic polymerization reaction is initiated. As the primer is elongated a new DNA strand containing modified bases is formed. The new DNA strand remains basepaired to the RNA template strand. D) The modified deoxyribonucleotide bases incorporated in the new DNA strand can be detected by several optional methods e.g. with an antibody directed against the modified deoxynucleotide labelled with a detectable label, such as a fluorophore, or conjugated with an enzyme that cleaves a substrate to generate a colorometric, fluorescent, chemiluminescent or electrochemical signal. The reverse transcriptase reaction can produce DNA products spanning multiple template lengths by a template switching mechanism.

In one aspect, the present invention relates to a kit of parts comprising a first ligand comprising a binding moiety, capable of specific binding to an analyte of interest, and a primer oligo-deoxyribonucleotide of a length of 18-40 nucleotides bound to the binding moiety; a template polyribonucleotide being longer than the primer oligo-deoxyribonucleotide and having a sequence that is at least partially complementary to the primer oligo-deoxyribonucleotide, a plurality of labelled deoxyribonucleotide triphosphates; and an enzyme with reverse transcriptase activity. Such kits are useful in performing the method according to the invention.

### EXAMPLES

### Materials

DynaLight^{™} Chemiluminescence AP substrate, ThermoFisher
LuMate^{®} Microplate Luminometer, Awareness Technology, Inc
HydroFlex^{™} microplate washer, Tecan.
Nunc^{™} NucleoLink^{™} Strips, white for luminescence. ThermoFisher
High binding white half-area microplates Bio-One Greiner
odT18, odT22 and odT30 (oligo-deoxy-thymidine) with NH2-C6 spacer arms synthesized at 1 µmol-scale, Eurofins.
Ab201796 Biotin (Type B) Fast Conjugation Kit, lot GR3353844-1, Abcam.
SoluLink Antibody-Oligonucleotide All-In-One Conjugation Kit, Abcam.
Streptavidin- alkaline phosphatase (AP) conjugate ThermoFisher
SuperSignal ELISA Pico Luminol/Enhancer solution and SuperSignal ELISA Pico Stable Peroxidase solution were purchased from ThermoFisher.
Streptavidin-HRP N100 ThermoFisher.
SARS-CoV-2 spike protein was prepared according to Rosendal *et al* 2020. The plasmid encoding the 2019-nCoV spike protein has been previously described, Wrapp, D. *et al.* 2020). Recombinant spike protein was produced using the 293F system, Thermo Fisher Scientific.

The protein was purified using a column packed with His-pure Ni-NTA resin, Thermo Fisher Scientific

### Human plasma samples

An anti-SARS-CoV-2 verification panel for serological assays was obtained from National Institute for Biological Standards and Control (NIBSC), Hertfordshire, UK. It is composed of 23 samples known to be anti-SARS-CoV-2 positive and 14 samples known to be anti-SARS-CoV-2 negative.

WHO International Standard for anti-SARS-CoV-2 immunoglobulin (human), NIBSC code: 20/136, was obtained from the same source.

### Polymerases:

Recombinant Moloney murine leukemia virus (M-MuLV) Reverse transcriptase (RT), New England BioLabs.

Recombinant Human immunodeficiency virus (HIV) 1 RT based on the BH10 sequence prepared according to Weber & Grosse (1989).
Klenow DNA polymerase, Large fragment, ThermoFisher
Recombinant human gamma polymerase (Pol-G) catalytic subunit, BioSite.
BST 3.0 DNA Polymerase , New England BioLabs.
IsoPol SD+, ArticZymes, Tromsø, Norway

### Antibodies and antibody preparations

Bu 20 mouse Monoclonal mouse anti-BrdU antibody conjugated to alkaline phosphatase (AP) (Code 09143) was purchased from Aglient/dako AB
Bovine anti-goat IgG conjugated with AP 805-055-180, Jackson Immuno Research
Rabbit anti-human IgG (H&L) A18905S, ThermoFisher
Rabbit anti-human IgG (H&L) AS10 1499, Agrisera
Goat anti-human IgG A18813, ThermoFisher .
Goat anti-human IgG Star 125, BioRad
IgG from human serum, reagent grade I-2511, Sigma-Aldrich
Mouse IgG, protein A purified, A66185M-LY, Meridian Life Science.
Goat IgG, purified, AS 10 875, Agrisera
The match pair mouse anti SARS-CoV-2 spike protein binding mabs DH6 / Ad10 was purchased from the Native Antigen Company, Oxford, UK
Mouse Bu20a-biotin (BioLegend 339810)

### Buffers used:

Carbonate buffer 0.05 M, pH 9.6
PBS-Tween: PBS med 0.05 % Tween 20
HIV RT Reaction solution: 10 mM Hepes pH 7.6, 32 µM BrdUTP, prA 100µg/ml, 4 mM MgCl₂, 2 mM spermine, Synperonic A11 0.5 %(v/v), 0.2 mM EGTA, and BSA 0.5 mg/ml .
HIV RT Reaction solution with IdUTP: As HIV RT Reaction solution above with 32 µM BrdUTP instead of 32 µM BrdUTP.
MMuLV RT reaction solution: 10 mM Hepes pH 7.6, 32 µM BrdUTP, prA 100 µg/ml, 6 mM MnCl₂, 24 mM spermidine, Triton X-100 0.5 %(v/v), 0.2 mM EDTA, 4 mM Glutathione and BSA 0.5 mg/ml
Pol-G reaction solution: 25 mM Hepes pH 8.0, 32 µM BrdUTP, prA 100 µg/ml, 0.5 mM MnCl₂, 100 mM NaCl, 1 mM Dithioerythritol I and BSA 0.66 mg/ml.
BST 3.0 DNA polymerase reaction solution: 25 mM TrisHCl pH 8.8, 32 µM BrdUTP, prA 100µg/ml, 8 mM MgSO₄, 150mM KCl, 0.1 %(v/v) Tween 20.
*Plate wash buffer*: 3 mM Boric acid, 0.75 % Triton X-100, 0.005 % (W/V) Dextran Sulphate, 0.2 % ETOH and 0.025 % NaN₃.
Blocking buffer: 1% non-fat dry-milk in PBS with 0.05 % Tween.
POLI reaction solution: 25 mM Hepes pH 8.0, 0.67 mg/ml BSA, 0.5 mM MnCl₂, 50 mM KCl, 0.5 mM Spermidine, 28 µg/ml PrA and 50 µM BrdUTP.
Blotto bulk: Non-fat dry milk 25 mg/ml, Bis-Tris 25 mM pH 7.0, K₂SO₄ 10 mM, Spermidine 1.0 mM, Dextran sulfat 1.0 1mg/ml, Tween 20 0.0.010 %, Dithioerythreitol 2.0 mM

### Methods

### Removal of mouse IgG cross-reacting material from rabbit anti-human IgG antibodies.

Mouse IgG was bound to NHS-agarose (GE Healthcare) according to the manufacturer's description, the gel was packed into a 2 ml column and washed with PBS. Rabbit anti-human IgG (H&L) Agrisera AS10 1499 in PBS (75 µg/ml) was loaded onto the column and the flow through was collected and stored at 4°C.

### Labelling of goat and rabbit anti-human IgG with biotin

Anti-human IgG was labeled with biotin using Ab201796 Biotin (Type B) fast Conjugation Kit, lot GR3353844-1 according to the manufacturer's description.

### Labelling of goat and rabbit anti-human IgG with odT22 or odT30.

Labelling with odT22 was done using SoluLink Antibody-Oligonucleotide All-In-One Conjugation Kit, Cat. No. A-9202-001 according to the manufacturer's description. 0.5 µmolel of NH2-C6-odT22 was used for tagging with formylbenzamide. 100 µl (^{~}55 µg) of affinity purified rabbit anti-human IgG Agrisera AS10 1499 was tagged with hydrazinonicotinamide. The two modified biomolecules were then mixed together in the presence of a reaction catalyst (aniline) to form the odT22 -IgG conjugate, which finally was purified using a magnetic affinity solid phase. Actual yield odT22-labeled IgG was 14 µg (200 µL at 67 µg/ml).

Labeling with odT30 was done using a modified protocol. 0.5 µmole of NH2-C6-odT30 was used for labeling 100 µg of affinity purified rabbit anti-human IgG Agrisera AS10 1499. The yield was 22 µg odT30-labeled IgG (200 µL at 110 µg/ml).

### Labelling of Ad10 mouse anti SARS-CoV-2 spike protein mabs with odT30.

Labeling of Ad10 with odT30 was done using a similar protocol as for the rabbit antihuman IgG. 0.125 µmole of NH2-C6-odT30 was used for labeling 100 µg of affinity purified Ad10 mouse anti SARS-CoV-2 spike protein mabs. The yield was 60 µg odT30-labeled IgG (200 µL at 300 µg/ml).

### Labeling of odT18, odT22 and odT30 with biotin.

odT18, odT22 and odT30 (oligo-deoxy-thymidine) with NH2-C6 spacer arms were labeled with biotin using Ab201796 Biotin (Type B) fast Conjugation Kit, lot GR3353844-1 according to the manufacturer's description

### Example 1 Basic polymerase boosted signal amplification model..

This example illustrates the capacity of the method according to the invention to detect and quantify an analyte. Oligonucleotide primers attached to antibodies or other types of affinity ligands are in the following examples used as label for analyte detection. After binding to their targets the oligonucleotides attached to the affinity ligands can serve as primers in a one-step polymerase reaction with a soluble complementary template and using labelled nucleotides. The so synthesized DNA strand is then detected using the incorporated labels.

To illustrate the concept of the invention, streptavidin is used as a model analyte. The first ligand capable of specific binding to the analyte consists of biotin as the moiety capable of specific binding to the analyte and odT22 as the primer oligo-deoxyribonucleotide. The template poly-ribonucleotide is prA. the labelled deoxyribonucleotides are Bromo-deoxy-Uridine-tri-phosphate (BrdUTP). The enzyme with reverse transcriptase activity is Moloney Murine Leukemia Virus (MMuLV) Reverse Transcriptase. The second ligand capable of specific binding to the labelled DNA strand is a conjugate consisting of an antibody part (a monoclonal mouse antibody with anti-BrdU specificity) and the enzyme alkaline phosphatase (AP).

### Immobilization of biotinylated odT22 on white Nunc^{™} NucleoLink^{™} strips plates

White NucleoLink^{™} strips mounted in a 96-wells frame were coated with 120 µl/well of 200 ng/ml streptavidin in carbonate buffer pH 9.6. Unbound streptavidin was removed by a careful washing procedure using the Hydroflex plate washer and plate wash buffer.

In the next step 120 µl of 5 times serial biotin-odT22 dilutions ranging from 1.7 x 10⁻¹⁶ to 1.0 x 10⁻²⁰ mol/well were added to the wells on the plate, allowed to bind, where-after the plate was washed again.

### Detection of bound odT22.

The bound odT can act as primers in a polymerase reaction. The enzymatic polymerization process used for primer detection is a Reverse Transcriptase (RT) reaction, whereby the enzyme builds a new DNA strand. To do this it needs an RNA template to copy, a short complementary DNA strand to start extending from (primer) and complementary deoxynucleotide(s) triphosphates that can be used as building blocks in the growing strand. The reaction is performed in this example on the 96-well plate (well volume 300 µl) with bound serial dilutions of biotin-odT22, wherein the odT22 serves as primer in the polymerization process.

The template complementary to the odT primer is prA, i.e. a single stranded polyriboadenylic acid composed of only one nucleotide base, adenine, that have an average chain length of approximately 500 nucleotides and are included in the RT reaction solution.

Also the other components needed for the RT reaction are part of the MMulv RT reaction mixture, which contains BrdUTP (Bromo-deoxy-Uridine-tri-phosphate). BrdUTP provides the nucleotide bases that are added to the primer by the recombinant M-Mulv RT, the enzyme performing the polymerization.

### Polymerase reaction

The polymerase reaction is started with the addition of 9.1 U/ well of MMuLV RT to each well on the plate, and the relative amount of the newly produced BrdU strand will be proportional to the amount of odT22 primers immobilized in the well.

The polymerase reaction is temperature dependent and reaction velocity increases with increasing temperature until enzyme function is impaired by denaturation. The microtiter plate is therefore placed on a heating block to provide a constant temperature of 37 C during the one hour incubation and also covered by a lid to prevent evaporation. Both the incubation temperature and the time for the reaction (incubation time) are factors that are to be controlled. The detection sensitivity in this system is a function of both the amount of polymerase added to each well and the reaction time.

The polymerase reaction is stopped after one hour by removing the RT-reaction solution. This is done by using the Hydroflex plate washer followed by a thorough wash procedure. The "plate wash buffer" used contains certain components e.g. detergent to increase the efficiency of the wash (see materials).

### Conjugate binding

After the reaction solution is washed away, an antibody-enzyme conjugate solution is added. The antibody part of the conjugate is a monoclonal mouse antibody with anti-BrdU specificity and the conjugate enzyme is alkaline phosphatase (AP). The conjugate binding reaction is also temperature dependent and the microtiter plate is therefore placed back on the heating block during the 37° C, 30 min incubation.

The conjugate binding step is ended by a careful wash procedure using the Hydroflex plate washer to remove the conjugate solution and non-specifically bound conjugate so that only conjugate bound to the BrdU remains. Increased wash temperature (30-35° C) is advantageous for a good wash result. The wash solution is the same as for the previous wash.

### Substrate reaction

In a final reaction, a solution containing DynaLight^{™} Chemiluminescence alkaline phosphatase (AP) substrate (ThermoFisher Scientific), is added to the wells. This substrate generates light when the AP enzyme part of the conjugate modifies the substrate structure. In this stage of the process the chemical reaction can be affected by airborne dust particles that may contain AP. Therefore after addition of substrate microtiter plate is moved directly into the Lumate Microplate Luminometer reading chamber where it is protected from dust. After a short "lag phase" of 10 minutes to obtain a stable light signal, the light intensity is measured by the luminometer.

The intensity of the light produced by the AP enzymatic process is proportional to the amount of conjugated enzyme bound to the well via the binding of the anti-BrdU antibody part to the synthesized BrdU strands. The amount of incorporated BrdU is in its turn proportional to the amount of odT primers that was present in a well.

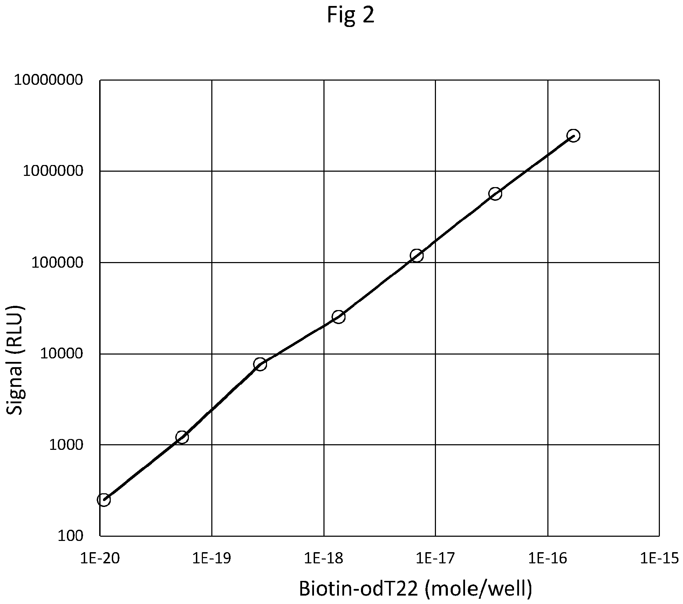

Fig.2 shows the relationship between the signal generated with polymerase boosted signal amplification and the amount of odT22 primer / well. As can be extracted from the figure odT amounts down to 1×10⁻²⁰ mol/well were detected and there was a linear relationship between amount of odT/well and the signal generated in the range from 1×10⁻²⁰ to 2x 10⁻¹⁶ mol odT/well. That is, a detection range of approximately 20 000 times.

### Example 2 Relationship between primer length and signal generated in the polymerase boosted signal amplification model.

The ability of oligonucleotide primers to function as primers for polymerases varies dependent on primer properties and reaction conditions. One crucial factor is primer length. Short primers are initially weakly associated to their templates and the initial polymerase reaction velocity is low until the elongated primer reaches a critical length. Increasing the primer length will lead to increased initial reaction velocity. Above a certain length, dependent on temperature and conditions, the primer will be permanently associated to the template. A further increase of the template length will not affect reaction velocity.

This has impact on the design of oligonucleotide primers in primers in polymerase boosted signal amplification. This example illustrates the relationship between primer length and generated signal.

*Immobilization of biotinylated odT 18, odT22 and odT30 on white Nunc^{™} NucleoLink^{™} strips plates* White NucleoLink^{™} strips mounted in a 96-wells frame were coated with 120 µl/well of 200 ng/ml streptavidin in carbonate buffer pH 9,6. Unbound streptavidin was removed by a careful wash procedure using the Hydroflex plate washer and plate wash buffer. In the next step 120 µl of indicated serial dilutions of biotin-odT 18, -odT22 and -odT30 were added, each to their own set of wells on the plate, allowed to bind, were after the plate was washed again.

### Polymerase reaction

The bound odT polymers act as primers in a polymerase reaction. The HIV reaction solution used contains everything essential for the reaction except primers and is described above in the Materials section. The polymerase reaction is started with the addition of 80 pg of HIV RT to each of the wells on the plate, and is allowed to proceed for 30 minutes at 37°C. The relative amounts of the newly produced BrdU strands will reflect the efficiency of the actual primer type and concentration to initiate and support an RT reaction.

The newly produced BrdU strand can be quantified utilizing AP-conjugated mouse Bu20 MAbs as outlined in the conjugate binding and substrate reaction sections of example 1.

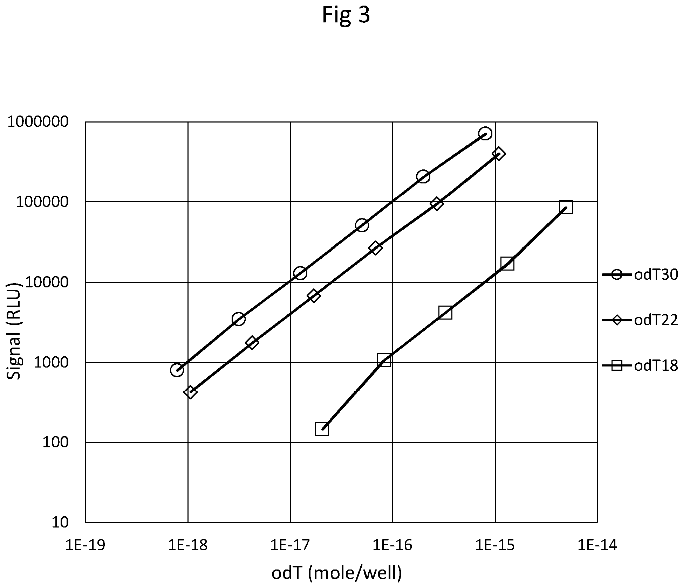

Fig.3 shows the relationship between the signal generated and the amounts of each indicated primer/ well. All three primer types were able to initiate an RT reaction. The highest signals encountered was generated by odT30, tightly followed by odT22, while odT18 produced significantly lower signals. The small difference between odT22 and odT30 primers indicate that their length are close to the optimal. Under the current reaction conditions approximatively 8×10⁻¹⁹ mol odT30 was enough to generate a signal of 1000 RLU, while approximately 100 times more odT18 was required to generate the same signal.

### Example 3. Use of eukaryotic, viral and bacterial polymerases in the polymerase boosted signal amplification step.

Different polymerases have different preferences for primer-template compositions. Reverse transcriptases use both RNA and DNA templates in vivo. Some DNA polymerases are strictly dependent on a DNA template whereas others may show polymerizing activity with RNA templates too or in other words act as reverse transcriptases. It is not clear if the reverse transcriptase activity plays an *in vivo* role or if it is just an *in vitro* phenomenon. The current invention use a polymerase to boost the signal that can be obtained from detection of an analyte . To this end it is advantageous to be able to choose among polymerases with different properties with respect to e.g. resistance to contaminants from the sample, processivity, reaction velocity, ability to make transcripts spanning several templates (template switching) and stability over a range of incubation times. This will provide more freedom to adapt the conditions for the polymerase boosted signal amplification step to the requirements of a particular assay for a particular analyte.

The current invention use antibodies or affinity ligands labelled with small oligonucleotide primers in the polymerase boosted signal amplification step. The complementary templates are free in the reaction solution. The ability to make transcripts spanning several templates is beneficial. The signal achieved varies with the amount of enzyme used. Polymerases with similar preferences are interchangeable. A slower reaction velocity can be compensated by the use of larger enzyme amounts. This example illustrate that either mammalian polymerase gamma (Pol G), HIV reverse transcriptase, the Klenow fragment of E.coli pol I DNA polymerase or another bacterial polymerase Bst 3.0 can be used in the polymerase boosted signal amplification step in an immunoassay with human IgG as analyte .

### Design of an immunoassay for human IgG

White NucleoLink^{™} strips mounted in a 96-wells frame were coated with 120 µl /well of human IgG diluted in PBS to 100 ng/ml. Unbound IgG was removed by a careful washing with plate wash buffer using a Hydroflex plate washer. In the next step 120 µl of biotinylated goat antihuman IgG at various dilutions were added, allowed to bind, whereafter the plate was washed again
30 µl of a mixture consisting of 10 ng / ml streptavidin and half the corresponding molar amount of biotin-odT22 was then added. After a brief incubation the plates were washed again.

Each well of the plate now contains three layers: First a layer of human IgG, the analyte, a second layer with serial dilutions of the ligand, biotininylated goat antihuman IgG and a third layer of streptavidin with bound odT22 primer. The amount of bound odT22 primers is proportional to the amount of bound ligand which is dependent on the amount of analyte. By applying the polymerase boosted signal amplification step the amount of analyte in a well can now be detected according to the invention

### Polymerase reaction

The sets of wells intended for HIV RT and Klenow DNA polymerase boosted signal amplification received 30 µl of aliquots HIV RT reaction solution, while the corresponding sets intended for pol G and Bst 3.0 polymerase boosted signal amplification received 30 µl aliquots of their respective reaction solutions. Each reaction solution contained all components required for a polymerase reaction except the odT primers. The polymerase reactions were started with the addition of 100 µl aliquots of enzyme for each set of wells. The HIV RT and Klenow DNA polymerase enzymes had been diluted to give approximatively the same enzymatic activity 8 U/ml Klenow fragment had an activity that corresponded to that of 800 pg/ml recombinant HIV RT. The pol G solution contained 2.4 µg enzyme /ml, the Bst 3.0 polymerase 0.16 U/ml.. The polymerase reactions were allowed to proceed for 30 minutes at 37 °C.

The polymerase reactions were stopped by removing the RT-reaction solution using the Hydroflex plate washer followed by a thorough wash procedure. Conjugate binding and substrate addition were performed as described in example 1.

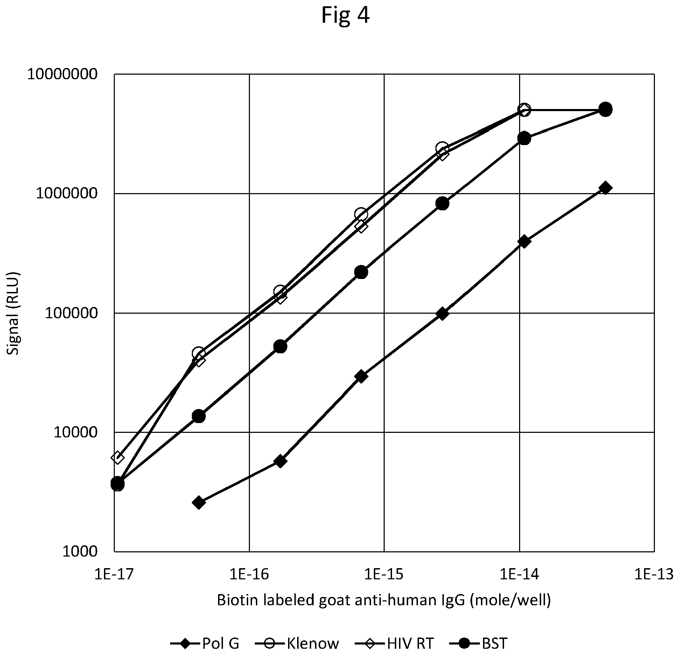

Fig. 4 shows shows the relationship between the signal generated by each of the four polymerases and the amounts biotinylated ligand added to the assay.

All four enzymes proved useful for polymerase boosted signal amplification. The HIV and Klenow polymerases had been diluted to give similar amounts of enzyme activity during the conditions used. As can be deduced from the figure, the two enzymes produced practically identical anti-human IgG titration profiles. The signals were, for all IgG concentrations, higher than the corresponding signal with pol G or Bst 3.0 polymerase due to use of higher enzyme input. Note that the signals generated by the HIV RT, Klenow fragment and Bst polymerases at the highest IgG concentration exceeded the reading range of the plate reader and thus deviated from linearity. See also example 5.

The pol G polymerase amount used gave a lower signal and it remained linear over the whole concentration range.

### Example 4 Relationship between signal generated by the polymerase boosted signal amplification step and amount of analyte and primer labeled ligand in the assay

Many classical immunoassays are designed to quantify the amount of immunoglobulin bound to a defined antigen. The detection sensitivity of the current invention is a function of several factors such as amounts of primer labeled antibodies or affinity ligands, primer length, amount and type of polymerase used, polymerase reaction conditions and reaction time and finally of the systems used for quantification of labeled DNA produced during the reaction.

### Design of immunoassay for human IgG

White NucleoLink^{™} strips mounted in a 96-wells frame were coated with 120 µl /well of indicated serial dilutions of human IgG diluted in PBS. Unbound IgG was removed by a careful washing with plate wash buffer using a Hydroflex plate washer. In the next step 120 µl containing either 25, 6.25 or 1.56 ng of odT22 conjugated rabbit anti-human IgG were added, allowed to bind for 30 min, whereafter the plate was washed again.

Each well of the plate now contains two layers: First a layer of serially diluted human IgG, the analyte, and a second layer with three dilutions of the ligand, odT22 conjugated rabbit antihuman IgG. The amount of bound odT22 primers is proportional to the amount of bound ligand which is dependent on the amount of analyte. By applying the polymerase boosted signal amplification step the amount of analyte in a well can now be detected and quantified according to the invention.

### Polymerase reaction

30 µl of a HIV RT reaction solution, containing all components for the polymerase reaction except odT primer, was added to each well on the plate. The polymerase reactions were then started with the addition of 120 µl of recombinant HIV 1 RT at 800 pg/ml. The polymerase reactions were allowed to proceed for 45 minutes at 37 °.

The polymerase reactions were stopped by removing the RT-reaction solution using the Hydroflex plate washer followed by a thorough wash procedure.

Conjugate binding and substrate addition were performed as described in example 1.

Fig. 5 illustrates the relationship between signal generated by the polymerase boosted signal amplification step and the amounts of human IgG antigen and odT22 conjugated anti-human IgG present in the assay.

The luminescence signals generated at different concentrations of odT22 labeled anti-human IgG antibody, were plotted towards the amount of human IgG immobilized in the wells. At each concentration of odT22 labelled antibody there was a linear relationship between amount of human IgG on the plate and the signal generated. Note that the system is far from saturated as regards to odT22 labeled antibody. Each increase in the amount of odT22 labeled antibody generated a proportionally increased signal and maximal detection sensitivity was not reached. Immobilized human IgG is detected at very low levels according to the invention. The detection range for e.g. the median concentration of odT22 labeled antibody in this experiment spanned from 5.3×10⁻¹⁸ to 1,7×10⁻¹⁴ moles human IgG / well, an approximately 3200 times range

### Example 5. Relationship between amount of polymerase used in the polymerase boosted signal amplification step and signal in an anti-SARS-CoV-2 spike protein antibody immunoassay.

As demonstrated in example 4, there is a linear relationship between the amounts of odT22 labeled ligand and the signal generated. There are many other factors affecting the signal level. The most obvious and most prominent are the amounts of enzyme activity used and the polymerization reaction time. The current example is intended to illustrate the use of the invention in an immunoassay measuring the antibody response to the SARS-CoV-2 spike protein after infection with the SARS-CoV-2 virus

### Design of an anti- SARS-CoV-2 spike protein antibody immunoassay

White NucleoLink^{™} strips mounted in a 96-wells frame were coated with 100 µl /well of SARS-CoV-2 spike protein, diluted in PBS to 100 ng/ml. Unbound spike protein was removed by a careful wash with PBS-Tween, using a Hydroflex plate washer. The coated plates were stored at -80 °C. Before use the wells were washed once with PBS-Tween and incubated for 1h at room temperature with blocking buffer, 1% non-fat dry-milk in PBS with 0.05 % Tween.

In the next step 100 µl samples from three sets of serial dilutions of heat-inactivated (56 °C for 1 hour) human sera positive for antibodies against SARS-Cov-2 spike protein were diluted in blocking buffer and added to the wells. The plate was incubated for 30 min and then washed in PBS-Tween buffer using a Hydroflex plate washer.

Then 30 µl of odT30 labelled rabbit antihuman IgG diluted in PBS-Tween to (50 ng/ml) was added. After a brief incubation the plate was washed again.

Each well of the plate now contains three layers: First a layer of SARS-COV-2 spike protein, the analyte, a second layer with serial dilutions of human plasmas containing anti-SARS-Cov-2 spike protein antibodies and a third layer with odT30 conjugated rabbit anti-human IgG. The amount of bound odT22 primers is proportional to the amount of bound human anti-spike antibody. By applying the polymerase boosted signal amplification step the presence of anti-spike antibodies in the plasma sample can now be detected and quantified according to the invention.

### Polymerase reaction

30 µl of a HIV RT reaction solution, containing all components for the polymerase reaction except odT primer, was added to each well on the plate. The polymerase reactions was then started by addition of 100 µl HIV 1 RT corresponding to either 80, 660 or 3290 pg HIV RT /well. The polymerase reactions were allowed to proceed for 45 minutes at 37 °C.

The polymerase reactions were stopped by removing the RT-reaction solution using the Hydroflex plate washer followed by a thorough wash procedure.

Conjugate binding and substrate addition were performed as described in example 1.

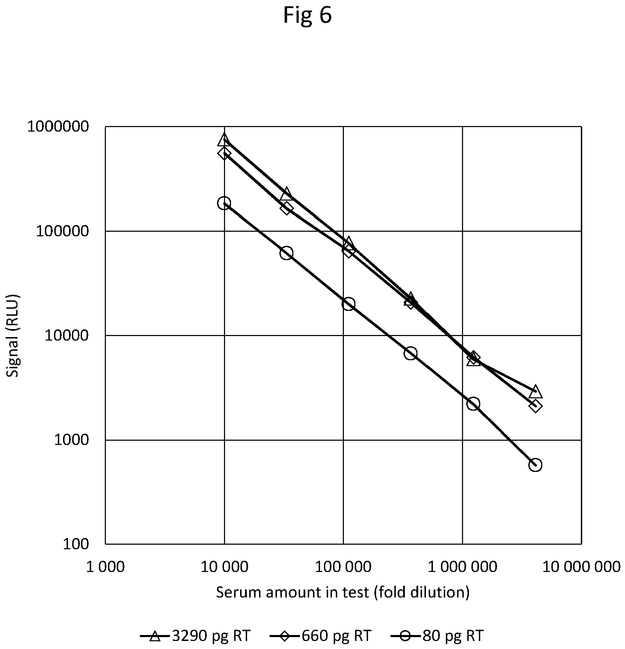

Fig. 6 illustrates how the detection sensitivity for this immunoassay for IgG antibodies to SARS-Cov-2 spike protein varied with the amount of polymerase used in the polymerase boosted signal amplification step. The polymerase reaction starts from the odT30 primers in the third layer of the immunoasay and proceeds on soluble templates added to the reaction solution. The signal generated in this system is proportional to the amount of BrdU strand produced and was found to increase approximately eight times when the amount HIV RT added increased from 80 to 660 pg RT /well. A further increase up to 3290 pg RT /well gave no or only a minor signal increase. When increasing the enzyme amount the system finally reach a saturated stage when all odT30 primer ends are occupied by polymerase at all times. As can be extracted from the figure this was valid at all serum dilutions.

### Example 6. Relationship between polymerase reaction time in the polymerase boosted signal amplification step and signal.

A normal enzyme reaction is linear with enzyme amount and reaction time as long as all the reaction components are present in excess. Due to practical reasons it is considered favourable to get the results from ligand binding assays within a few hours. The polymerase reaction time have thus in examples 1-5 been restricted to 30-60 minutes. The present invention, however, provides the possibility to gain increased detection sensitivity by increasing the polymerase reaction time. The primer extension step in the current invention is based on the combination of primer labeled ligands bound to solid phases and free templates in the reaction solution. The ability to sequentially utilize several templates to perform polymerization across template borders is beneficial for avoiding that access to templates becomes rate limiting for the polymerase reaction in the signal amplification step.

### Immobilization of odT22 on white Nunc^{™} NucleoLink^{™} strips plates

White NucleoLink^{™} strips mounted in a 96-wells frame were coated with 120 µl/well of 200 ng/ml streptavidin in carbonate buffer pH 9.6. Unbound streptavidin was removed by a careful wash procedure using the Hydroflex plate washer and plate wash buffer. In the next step 120 µl of 3.125 pg biotin-odT22 /ml and were added to the wells on the plate, allowed to bind, were after the plate was washed again.

### Polymerase reaction

The streptavidin-odT22 coated strips were transferred to six separate frames, two strips at each. One strip on each frame received 130 µl of a HIV RT reaction solution containing 800 pg HIV1 RT /ml, the other a MMuLV RT reaction solution containing 0.2 U MMuLV RT /ml. Both reaction solutions contained everything essential for the polymerase reaction except primers and are described in materials. The frames with strips were transferred to a 37 °C incubator. One frame was removed from the incubator after 30 minutes and frozen at -20°C. Another frame was removed from the incubator after 60 minutes and processed as the first. This process was repeated after 120, 240, 355 and 1090 minutes. When the last frame was frozen all strips were assembled into one frame., After thawing, the polymerase reactions were stopped by removing the RT-reaction solution using the Hydroflex plate washer followed by a thorough wash procedure.

Conjugate binding and substrate addition were performed as described in example 1.

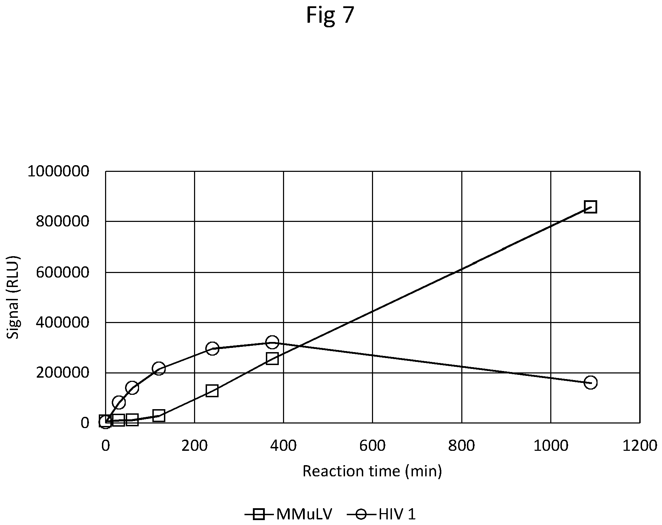

Fig. 7 show the signal development in relation to the polymerase reaction time in the polymerase boosted signal amplification step when using either HIV-1 or MMuLV RT. Both enzymes generated increasing signals for several hours, but behaved strikingly different during long incubation times. The MMuLV enzyme was a "slow starter" but sustained a polymerase reaction that was linear with time for many hours after the initial "lag phase". The HIV-1 RT, on the other hand, started immediately to produce labelled DNA, but the reaction velocity decreased after a few hours and the reaction even appeared to reverse after approximately 400 minutes.

A linear polymerase reaction for a few hours requires that the new DNA strands are much longer than the templates of approximately 500 bases. The long RNA/DNA strands produced are at certain reaction conditions obviously sensitive to nucleases. The relationship between time and signal generated varies between different combinations of enzymes and reaction mixtures. For each defined application, this has to be taken into account when designing an immunoassay containing the polymerase boosted signal amplification step according to the invention.

### Example 7. Effect on the signal of using two different deoxyuridine triphosphate analogues in the signal amplification step in an anti-SARS-CoV-2 spike protein antibody immunoassay

The polymerase boosted signal amplification step in examples 1-6 is based on use of one deoxythymidine triphosphate analogue, BrdUTP, to label newly synthesized DNA. It is obviously possible to use other nucleotide analogues and detect them with similar methods. The signal will depend both on the reaction velocity for incorporation of the deoxythymidine triphosphate analogue and the efficiency of the detection of the analogue in the newly synthesized DNA. In the current example we compare the signals generated by IdUTP and BrdUTP as two different modified deoxyuridine triphosphate analogues used in the polymerase boosted signal amplification step in an anti- SARS-CoV-2 spike protein antibody immunoassay

### Design of an anti- SARS-CoV-2 spike protein antibody immunoassay

High binding white half-area microplates from Bio-One were coated with SARS-CoV-2 spike protein, as described in example 5.

In the next step serial dilutions of two heat-inactivated (56 °C for 1 hour) human sera were produced, one anti-SARS-CoV-2 spike protein antibody positive (UK 9) and one antibody negative (UK 32) both diluted in blocking buffer. They were added to wells on two separate plates. The plates were incubated for 30 min and then washed in PBS-Tween buffer using a Hydroflex plate washer. Then 30 µl of odT30 conjugated rabbit anti-human IgG diluted in PBS-Tween to 200 ng/ml was added. After a brief incubation the plate was washed again.

### Polymerase reaction

One of the two plates with identical serum titrations received 30 µl/ well of a HIV RT reaction solution, containing BrdUTP and all other components for the polymerase reaction except the odT primer. The remaining plate received 30 µl of an identical HIV RT reaction solution but with IdUTP instead of BrdUTP. The polymerase reactions was then started with the addition of HIV 1 RT (240 pg) to each well on the plates. The polymerase reactions were allowed to proceed for 30 minutes at 37 °C. The reaction was stopped by removing the RT-reaction solution using the Hydroflex plate washer followed by a thorough wash procedure.

### Conjugate binding and substrate addition were performed as described in example 1

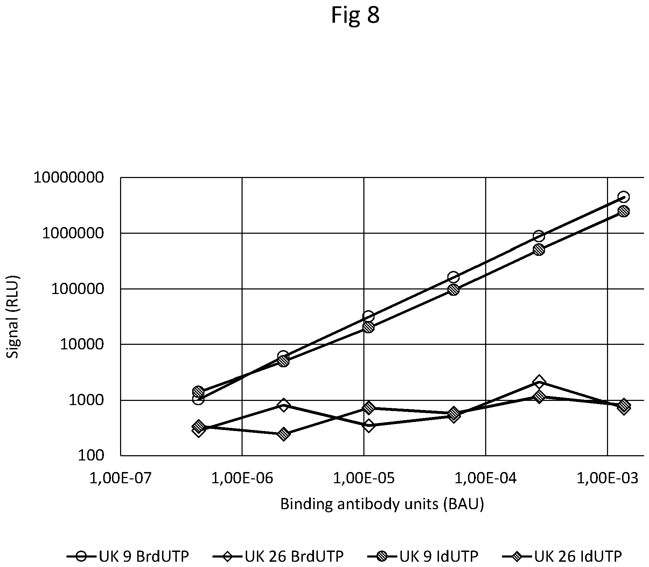

Fig. 8 illustrates the use of two different modified deoxyuridine triphosphate analogues in the polymerase boosted signal amplification step in an immunoassay for detection of human IgG antibodies against the SARS-CoV-2 spike protein. The positive serum UK 9 from the anti SARS-CoV-2 verification panel from NIBSC was calibrated towards the WHO international standard for anti SARS-CoV-2 immunoglobulin (NIBSC Code 20/136) and the amount of UK 9 in the figure is expressed as IgG antibody binding units (BAU). The negative serum UK 26 from the same panel was included for comparison in the figure at the same dilutions as the positive UK9 serum.

BrdUTP as substrate for HIV RT generated slightly higher signals compared to IdUTP. The span for linear detection was found to be similar for both analogues and ranged from 0,4 to 1300 µBAU. The immunoassay with the polymerase boosted signal amplification step proved to be able to differentiate between the SARS-CoV-2 positive (UK9 ) serum and the negative serum (UK26) at dilutions down to 0,4 µ BAU.

### Example 8. A polymerase boosted antigen capture ELISA for SARS-CoV-2 spike protein.

Utilization of the invention to booster assays quantifying antibodies binding to immobilized antigens have been demonstrated in examples 5 and 7. The current example is intended to illustrate the use of the invention to booster an antigen capture assay. Both signal and detection range in ELISA varies with the amount and type of label on the detection antibody. In examples 1-to 7 we have used AP conjugated monoclonal antibodies (mabs) for this purpose. The current example is thus also intended to demonstrate the usability of other enzyme labels, such as horseradish peroxidase (HRP), in polymerase boosted ELISA.

### Design of first layer in a SARS-CoV-2 spike protein ELISA

Microtiter plates, consisting of white Nunc Nucleolink strips were coated with 100 µl/well of DH6 mouse anti SARS-CoV-2 spike protein mabs in PBS (500 ng/ml). The plates were covered with sealing tape and incubated in fridge overnight. Unbound mabs were removed using the Hydroflex plate washer and PBS Tween. The coated plates were incubated for 1h at 37°C with blocking buffer (1% non-fat dry-milk in PBS). The plates were then washed again.

### Adding a second and third layer.

In the next step 20 µl of Ad10 mouse anti SARS-CoV-2 spike protein mabs labelled with odT30 in blocking buffer with 0.5% triton X-100 (1000 ng/ml) was added to each well on the plate. Then 80 µl samples and blanks from a set of serial dilutions of SARS-Cov-2 spike protein were added (starting with 12.5 ng/ml and serially diluted in two steps in blocking buffer with 0.5% triton X-100 until reaching 6.1 pg/ml). After a 60 min incubation the plate was washed again.

Each well of the plate now contain a third layer with odT30 labelled mouse anti SARS-Cov-2 spike protein. The amount of bound odT30 primers is proportional to the amount of SARS-Cov-2 spike protein in the second layer and can be quantified according to the invention.

### Polymerase reaction

30 µl of a POLI reaction solution, containing all components for the polymerase reaction except odT primers, was added to each well on the plate. The polymerase reactions were then started by addition of 12.5 µU IsoPol SD+ in 100 µl 25 mM hepes, pH 8.0 with 0.67 mg BSA/ ml The polymerase reactions were allowed to proceed for 60 minutes at room temperature.

The polymerase reactions was stopped by removing the RT-reaction solution using the Hydroflex plate washer.

### Quantification of newly produced BrdU strand

In this example we utilize a two-step conjugate binding procedure. 100 µl biotin conjugated mouse Bu20 mabs (0.6 µg/ml in blotto bulk) was first added to each well. After a brief incubation the plate was washed and 100 µl HRP labeled Streptavidin (0.42 µg/ml in PBS Tween with 0.1% casein) was added. Each well on the plate will now contain a HRP labeled biotin-streptavidin complex bound to the newly synthesized BrdU strand.

### Substrate reaction

6.1 ml SuperSignal ELISA Pico Luminol/Enhancer solution was mixed with 6.1 ml SuperSignal ELISA Pico Stable Peroxidase solution to get a HRP substrate working solution. In a final reaction, 120 µl of this solution is added to each well. This substrate generates light when the HRP enzyme part of the conjugate modifies the substrate structure. The RT reaction plate was moved into the Lumate Microplate Luminometer reading chamber and the light intensity was measured.

The intensity of the light produced by the HRP enzymatic process is proportional to the amount of conjugated enzyme bound to the well via the binding of the anti-BrdU antibody to the synthesized BrdU strands. The amount of incorporated BrdU is in its turn proportional to the amount of odT primers on the Ad10 mabs bound to the SARS-CoV-2 spike protein captured in the wells.

### The reference ELISA

The same components, at the same concentrations were used as in the polymerase boosted assay. The AD10 detection antibody in the reference ELISA was labeled with biotin, not odT. The presence of this of this antibody was subsequently detected directly with HRP labeled streptavidin, without any signal amplification step.

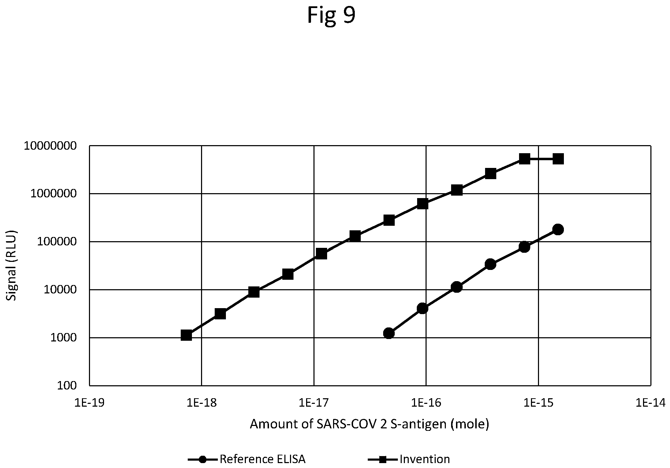

Fig. 9 illustrates the relationship between signals generated by either a polymerase boosted antigen capture assay or a reference ELISA and the amount of SARS-CoV-2 spike protein present.

As can be extracted from the figure the polymerase boosted capture assay produced a linear relationship between signal generated and amount of spike protein present ranging from approximately 7×10⁻¹⁹ to 1×10⁻¹⁵ mole. The deviation from linearity close to upper detection limit was due to limitations in the detection range of the luminometer.

The reference ELISA was not able to detect spike protein levels below 5×10⁻¹⁷ moles.

### References

Collins E, Rothacker JA. Methods of detecting an analyte in a sample. WO2006/053380.
Hendrickson ER, Haffield Truby TM, Joerger RD, et al.. High sensitivity multianalyte immunoassay using covalent DNA-labeled antibodies and polymerase chain reaction., Nucleic Acids Research, 1995, Vol 23, No.3, 522-529.
Huber HE, McCoy JM, Seehra JS, Richardson CC. Human immunodeficiency virus 1 reverse transcriptase. Template binding, processivity, strand displacement synthesis, and template switching. J Biol Chem, 1989, 264(8): 4669-78. PMID: 2466838.
Kralik P & Ricchi M. A Basic Guide to Real Time PCR in Microbial Diagnostics: Definitions, Parameters, and Everything. Frontiers in Microbiology, 2017, 8, 108- DOI={10.3389/fmicb.2017.00108}
Lannergren U. Methods and compositions for universal size specific PCR. US 2005/0233351A
Lennerstrand J, Rytting AS, Örvell C et al. A method for combined immunoaffinity purification and assay of HIV-1 reverse transcriptase activity useful for crude samples. Anal Biochem, 1996 Mar 15; 235(2): 141-52. doi: 10.1006/abio.1996.0106. PMID: 8833322.
Petterson I, Shao X, Gronowitz S, Källander C. (2001) A method for measuring DNA polymerisation and applications of the method. PCT/SE02/01155 (WO 02/103039 A1)
Rosendal E, Wigren Byström J, Gröning R et al. Detection of asymptomatic SARS-CoV-2 exposed individuals by a sensitive S-based ELISA. BMJ medRxiv preprint doi: doi.org/10.1101/2020.06.02.20120477.this version posted June 4, 2020.
Sano T, Smith CL and. Cantor CR, "Immuno-PCR: Very Sen-sitive Antigen Detection by Means of Specific Anti-body-DNA Conjugates." Science, 1992, 258,120-122.
Sano T; Cantor CR; Smith CL. Immuno-polymerase chain reaction system for antigen detection. US 5,665,539, 1993
Simonova MA, Pivovarov VP, Ryazantsev DY et al.. Comparative diagnostics of allergy using quantitative immuno-PCR and ELISA. Bioanalysis, 2018; 10(10): 757-767. doi: 10.4155/bio-2017-0194. Epub 2018 May 17.
Todd J, Freese B, Lu A, Held D, Morey J, Livingston R, Goix P. Ultrasensitive flow-based immunoassays using single-molecule counting. Clin Chem. 2007 Nov;53(11):1990-5. doi: 10.1373/clinchem.2007.091181. Epub 2007 Sep 21. PMID: 17890441.
Urdea M. Protein-nucleic acid probes and immunoassays using same. 1991 WO 91/17442.
Van Dessel W, Vandenbussche F, Staes M, Goris N, De Clercq K. Assessment of the diagnostic potential of immuno-RCA in 96-well ELISA plates for foot-and-mouth disease virus. J Virol Methods. 2008 Jan;147(1):151-6. doi: 10.1016/j.jviromet.2007.08.020. Epub 2007 Oct 25. PMID: 17913251.
Weber J, Grosse F. Fidelity of human immunodeficiency virus type I reverse transcriptase in copying natural DNA. Nucleic Acids Res. 1989 Feb 25;17(4):1379-93. doi: 10.1093/nar/17.4.1379. PMID: 2466238; PMCID: PMC331810.
Wei H, Wang E. Electrochemiluminescence of tris(2,2'-bipyridyl)ruthenium and its applications in bioanalysis: a review. Luminescence. 2011 Mar-Apr;26(2):77-85. doi: 10.1002/bio.1279. Epub 2011 Mar 14. PMID: 21400654.
Wrapp D. et al. Cryo-EM structure of the 2019-nCoV spike in the prefusion conformation. Science 367, 1260-1263, doi:10.1126/science.abb2507 (2020).
Zhou Y, Calciano M, Hamann S, Leamon JH, Strugnell T, Christian MW, Lizardi PM. In situ detection of messenger RNA using digoxigenin-labeled oligonucleotides and rolling circle amplification. Exp Mol Pathol. 2001 Jun;70(3):281-8. doi: 10.1006/exmp.2001.2365. PMID: 11418007.

## Claims

1. A method for detection of an analyte of interest in a sample comprising
- bringing a first ligand capable of specific binding to the analyte in contact with the sample under conditions facilitating specific binding of the ligand to the analyte, wherein the first ligand comprises a binding moiety capable of specific binding to the analyte and a primer oligo-deoxyribonucleotide bound to the binding moiety wherein the primer oligo-deoxyribonucleotide has a length of 18-40 nucleotides;
- removing unbound first ligand from the sample;
- bringing the bound first ligand in contact with a template polyribonucleotide being longer than the primer oligo-deoxyribonucleotide and having a sequence that is at least partially complementary to the primer oligo-deoxyribonucleotide; a plurality of labelled deoxyribonucleotide triphosphates; and an enzyme with reverse transcriptase activity;
- incubating the bound first ligand comprising the attached primer oligo-deoxyribonucleotide, the template polyribonucleotide, the labelled deoxyribonucleotide triphosphates, and the enzyme under conditions suitable for synthesis of a labelled DNA-strand by said enzyme; and
- detecting any synthesized labelled DNA strand.

2. The method according to claim 1, wherein the primer oligo-deoxyribonucleotide is selected from oligo-deoxyribonucleotides containing all four bases, A,G,T and C or a subset thereof or from oligo-deoxyribonucleotides containing a single base, preferably oligo-deoxythymidine.

3. The method according to claim 2, wherein the labelled deoxyribonucleotides are labelled deoxyuridine triphosphates (dUTP), such as bromo-dUTP or iodo-dUTP, preferably bromo-dUTP, and the template polyribonucleotide is polyriboadenylic acid.

4. The method according to any one of claims 1-3, wherein the template polyribonucleotide has a length of 10²-10³ nucleotides, preferably 2*10²-5*10² nucleotides.

5. The method according to any one of claims 1-4, wherein the enzyme with reverse transcriptase activity is a eukaryotic polymerase, such as DNA polymerase gamma; a viral polymerase, such as a retroviral reverse transcriptase, preferably HIV or M-MuLV reverse transcriptase, or a bacterial polymerase, such as a bacterial polymerase is derived from mesophilic bacteria, preferably the Klenow fragment of *E.coli* DNA polymerase I , or a bacterial polymerase derived from thermophilic bacteria, preferably Bst 3.0 derived from *Bacillus Stearothermophilus.*

6. The method according to any one of claims 1-5 wherein the incubation step is performed at a constant temperature, preferably at 37 °C, for 30 min-4hrs, preferably for 1hr.

7. The method according to any one of claims 1-6, wherein the detection is performed through a detection method comprising the steps
- bringing a second ligand capable of specific binding to the labelled DNA strand in contact with the synthesized labelled DNA strand under conditions facilitating specific binding of the second ligand to the DNA strand, wherein the second ligand comprises a binding moiety capable of specific binding to the synthesized labelled DNA and a covalently attached enzyme;
- removing the unbound second ligand from the sample;
- adding a substrate for the attached enzyme that upon cleavage by the attached enzyme generates a detectable signal; and
- detecting said signal.

8. The method according to claim 7, wherein said second ligand is an antibody which specifically binds to the synthesized labelled DNA having covalently attached thereto an enzyme, such as alkaline phosphatase or horse radish peroxidase.

9. The method according to claim 8, wherein the covalently attached enzyme acts on a substrate to generate a colorimetric, fluorescent, chemiluminescent or electrochemical signal

10. The method according to any one of the preceding claims, wherein the detection of the analyte of interest is a quantitative detection.

11. A kit of parts comprising a first ligand comprising a binding moiety, capable of specific binding to an analyte of interest, and a primer oligo-deoxyribonucleotide of a length of 18-40 nucleotides bound to the binding moiety; a template polyribonucleotide being longer than the primer oligo-deoxyribonucleotide and having a sequence that is at least partially complementary to the primer oligo-deoxyribonucleotide, a plurality of labelled deoxyribonucleotide triphosphates; and an enzyme with reverse transcriptase activity.

12. The kit of parts according to claim 11, wherein the primer oligo-deoxyribonucleotide is selected from oligo-deoxyribonucleotides containing all four bases, A,G,T and C or a subset thereof or from oligo-deoxyribonucleotides containing a single base, preferably oligo-deoxythymidine.

13. The kit of parts according to any one of claims 11-12, wherein the labelled deoxyribonucleotide triphosphates are labelled deoxyuridine triphosphate (dUTP), such as bromo-dUTP or iodo-dUTP, preferably bromo-dUTP, and the template polyribonucleotide is polyriboadenylic acid.

14. The kit of parts according to any one of claims 11-13 wherein the template poly-ribonucleotide has a length of 10²-10³ nucleotides, preferably 2*10²-5*10² nucleotides.

15. The kit of parts according to any one of claims 11-14, wherein the enzyme with reverse transcriptase activity is a eukaryotic, viral or bacterial polymerase, such as DNA polymerase gamma, HIV or M-MuLV reverse transcriptase, the Klenow fragment of *E.coli* DNA polymerase I or Bst 3.0 derived from *Bacillus Stearothermophilus.*

16. The kit of parts according to any one of claims 11-15, further comprising a second ligand capable of specific binding to the labelled deoxyribonucleotides, said second ligand also comprising a covalently attached enzyme; and a substrate for said enzyme.

17. The kit of parts according to claim 16, wherein said second ligand is a monoclonal antibody having covalently attached thereto an enzyme, such as alkaline phosphatase or horse radish peroxidase

## Patentansprüche

1. Verfahren zum Nachweis eines Analyten von Interesse in einer Probe, umfassend
- Inkontaktbringen eines ersten Liganden, der in der Lage ist, spezifisch an den Analyten zu binden, mit der Probe unter Bedingungen, die eine spezifische Bindung des Liganden an den Analyten fördern, wobei der erste Ligand eine Bindungsgruppe, die in der Lage ist, spezifisch an den Analyten zu binden, und ein Primer-Oligo-Desoxyribonukleotid, das an die Bindungsgruppe gebunden ist, umfasst, wobei das Primer-Oligo-Desoxyribonukleotid eine Länge von 18-40 Nukleotiden aufweist;
- Entfernen von ungebundenem erstem Liganden aus der Probe;
- Inkontaktbringen des gebundenen ersten Liganden mit einem Matrizen-Polyribonukleotid, das länger als das Primer-Oligo-Desoxyribonukleotid ist und eine Sequenz aufweist, die mindestens teilweise komplementär zu dem Primer-Oligo-Desoxyribonukleotid ist; einer Vielzahl von markierten Desoxyribonukleotid-Triphosphaten; und einem Enzym mit reverser Transkriptase-Aktivität;
- Inkubieren des gebundenen ersten Liganden, der das verknüpfte Primer-Oligo-Desoxyribonukleotid, das Matrizen-Polyribonukleotid, die markierten Desoxyribonukleotid-Triphosphate und das Enzym umfasst, unter Bedingungen, die zur Synthese eines markierten DNA-Strangs durch das Enzym geeignet sind; und
- Nachweisen jeglichen synthetisierten markierten DNA-Strangs.

2. Verfahren nach Anspruch 1, wobei das Primer-Oligo-Desoxyribonukleotid aus Oligo-Desoxyribonukleotiden, die alle vier Basen, A, G, T und C, oder eine Teilmenge davon enthalten, oder aus Oligo-Desoxyribonukleotiden, die eine einzige Base enthalten, vorzugsweise Oligo-Desoxythymidin, ausgewählt ist.

3. Verfahren nach Anspruch 2, wobei die markierten Desoxyribonukleotide markierte Desoxyuridin-Triphosphate (dUTP), wie etwa Brom-dUTP oder Iod-dUTP, vorzugsweise Brom-dUTP, sind und das Matrizen-Polyribonukleotid Polyriboadenylsäure ist.

4. Verfahren nach einem der Ansprüche 1-3, wobei das Matrizen-Polyribonukleotid eine Länge von 10²-10³ Nukleotiden, vorzugsweise 2*10²-5*10² Nukleotiden aufweist.

5. Verfahren nach einem der Ansprüche 1-4, wobei das Enzym mit reverser Transkriptase-Aktivität eine eukaryotische Polymerase ist, wie etwa DNA-Polymerase gamma; eine virale Polymerase, wie etwa eine retrovirale reverse Transkriptase, vorzugsweise HIV- oder M-MuLV-reverse Transkriptase, oder eine bakterielle Polymerase, wie etwa eine bakterielle Polymerase, die von mesophilen Bakterien abgeleitet ist, vorzugsweise das Klenow-Fragment von *E.coli*-DNA-Polymerase I, oder eine bakterielle Polymerase, die von thermophilen Bakterien abgeleitet ist, vorzugsweise von *Bacillus stearothermophilus* abgeleitete Bst 3.0.

6. Verfahren nach einem der Ansprüche 1-5, wobei der Inkubationsschritt bei einer konstanten Temperatur, vorzugsweise bei 37 °C, für 30 min - 4 h, vorzugsweise für 1 h, durchgeführt wird.

7. Verfahren nach einem der Ansprüche 1-6, wobei der Nachweis mittels eines Nachweisverfahrens durchgeführt wird, das die Folgenden Schritte umfasst:
- Inkontaktbringen eines zweiten Liganden, der in der Lage ist, spezifisch an den markierten DNA-Strang zu binden, mit dem synthetisierten markierten DNA-Strang unter Bedingungen, die eine spezifische Bindung des zweiten Liganden an den DNA-Strang fördern, wobei der zweite Ligand eine Bindungsgruppe, die in der Lage ist, spezifisch an die synthetisierte markierte DNA zu binden, und ein kovalent verknüpftes Enzym umfasst;
- Entfernen des ungebundenen zweiten Liganden aus der Probe;
- Zugeben eines Substrats für das verknüpfte Enzym, das bei Spaltung durch das verknüpfte Enzym ein nachweisbares Signal erzeugt; und
- Nachweisen des Signals.

8. Verfahren nach Anspruch 7, wobei der zweite Ligand ein Antikörper ist, der spezifisch an die synthetisierte markierte DNA bindet, die ein kovalent damit verknüpftes Enzym, wie etwa alkalische Phosphatase oder Meerrettich-Peroxidase, aufweist.

9. Verfahren nach Anspruch 8, wobei das kovalent verknüpfte Enzym auf ein Substrat einwirkt, um ein kolorimetrisches, fluoreszierendes, chemilumineszierendes oder elektrochemisches Signal zu erzeugen.

10. Verfahren nach einem der vorhergehenden Ansprüche, wobei der Nachweis des Analyten von Interesse ein quantitativer Nachweis ist.

11. Kit von Teilen, umfassend einen ersten Liganden, der eine Bindungsgruppe, die in der Lage ist, spezifisch an einen Analyten von Interesse zu binden, und ein Primer-Oligo-Desoxyribonukleotid mit einer Länge von 18-40 Nukleotiden, das an die Bindungsgruppe gebunden ist, umfasst; ein Matrizen-Polyribonukleotid, das länger als das Primer-Oligo-Desoxyribonukleotid ist und eine Sequenz aufweist, die mindestens teilweise komplementär zu dem Primer-Oligo-Desoxyribonukleotid ist, eine Vielzahl von markierten Desoxyribonukleotid-Triphosphaten; und ein Enzym mit reverser Transkriptase-Aktivität.

12. Kit von Teilen nach Anspruch 11, wobei das Primer-Oligo-Desoxyribonukleotid aus Oligo-Desoxyribonukleotiden, die alle vier Basen, A, G, T und C, oder eine Teilmenge davon enthalten, oder aus Oligo-Desoxyribonukleotiden, die eine einzige Base enthalten, vorzugsweise Oligo-Desoxythymidin, ausgewählt ist.

13. Kit von Teilen nach einem der Ansprüche 11-12, wobei die markierten Desoxyribonukleotid-Triphosphate markiertes Desoxyuridin-Triphosphat (dUTP), wie etwa Brom-dUTP oder Iod-dUTP, vorzugsweise Brom-dUTP, sind und das Matrizen-Polyribonukleotid Polyriboadenylsäure ist.

14. Kit von Teilen nach einem der Ansprüche 11-13, wobei das Matrizen-Polyribonukleotid eine Länge von 10²-10³ Nukleotiden, vorzugsweise 2*10²-5*10² Nukleotiden aufweist.

15. Kit von Teilen nach einem der Ansprüche 11-14, wobei das Enzym mit reverser Transkriptase-Aktivität eine eukaryotische, virale oder bakterielle Polymerase ist, wie etwa DNA-Polymerase gamma, HIV- oder M-MuLV-reverse Transkriptase, das Klenow-Fragment von *E.coli*-DNA-Polymerase I oder von *Bacillus stearothermophilus* abgeleitete Bst 3.0.

16. Kit von Teilen nach einem der Ansprüche 11-15, ferner umfassend einen zweiten Liganden, der in der Lage ist, spezifisch an die markierten Desoxyribonukleotide zu binden, wobei der zweite Ligand zudem ein kovalent verknüpftes Enzym umfasst; und ein Substrat für das Enzym.

17. Kit von Teilen nach Anspruch 16, wobei der zweite Ligand ein monoklonaler Antikörper ist, der ein kovalent damit verknüpftes Enzym, wie etwa alkalische Phosphatase oder Meerrettich-Peroxidase, aufweist.

## Revendications

1. Procédé de détection d'un analyte d'intérêt dans un échantillon comprenant :
- la mise en contact d'un premier ligand capable de se lier spécifiquement à l'analyte avec l'échantillon dans des conditions facilitant la liaison spécifique du ligand à l'analyte, le premier ligand comprenant une fraction de liaison capable de se lier spécifiquement à l'analyte et un oligo-désoxyribonucléotide d'amorce lié à la fraction de liaison, l'oligo-désoxyribonucléotide d'amorce ayant une longueur de 18 à 40 nucléotides ;
- l'élimination du premier ligand non lié de l'échantillon ;
- la mise en contact du premier ligand lié avec un polyribonucléotide matrice plus long que l'oligo-désoxyribonucléotide d'amorce et ayant une séquence qui est au moins partiellement complémentaire de l'oligo-désoxyribonucléotide d'amorce ; une pluralité de désoxyribonucléotides triphosphates marqués ; et une enzyme ayant une activité de transcriptase inverse ;
- l'incubation du premier ligand lié comprenant l'oligo-désoxyribonucléotide d'amorce attaché, le polyribonucléotide matrice, les désoxyribonucléotides triphosphates marqués et l'enzyme dans des conditions appropriées pour la synthèse d'un brin d'ADN marqué par ladite enzyme ; et
- la détection de tout brin d'ADN marqué synthétisé.

2. Procédé selon la revendication 1, dans lequel l'oligo-désoxyribonucléotide d'amorce est choisi parmi les oligo-désoxyribonucléotides contenant les quatre bases, A, G, T et C ou un sous-ensemble de celles-ci ou parmi les oligo-désoxyribonucléotides contenant une seule base, de préférence l'oligo-désoxythymidine.

3. Procédé selon la revendication 2, dans lequel les désoxyribonucléotides marqués sont des désoxyuridine triphosphates marqués (dUTP), tels que le bromo-dUTP ou l'iodo-dUTP, de préférence le bromo-dUTP, et le polyribonucléotide matrice est l'acide polyriboadénylique.

4. Procédé selon l'une quelconque des revendications 1 à 3, dans lequel le polyribonucléotide matrice a une longueur de 10² à 10³ nucléotides, de préférence de 2*10² à 5*10² nucléotides.

5. Procédé selon l'une quelconque des revendications 1 à 4, dans lequel l'enzyme ayant une activité de transcriptase inverse est une polymérase eucaryote, telle que l'ADN polymérase gamma ; une polymérase virale, telle qu'une transcriptase inverse rétrovirale, de préférence la transcriptase inverse du VIH ou du M-MuLV, ou une polymérase bactérienne, telle qu'une polymérase bactérienne dérivée de bactéries mésophiles, de préférence le fragment de Klenow de l'ADN polymérase I d'*E. coli,* ou une polymérase bactérienne dérivée de bactéries thermophiles, de préférence Bst 3.0 dérivée de *Bacillus Stearothermophilus.*

6. Procédé selon l'une quelconque des revendications 1 à 5, dans lequel l'étape d'incubation est réalisée à une température constante, de préférence à 37 °C, pendant 30 min à 4 h, de préférence pendant 1 h.

7. Procédé selon l'une quelconque des revendications 1 à 6, dans lequel la détection est réalisée par un procédé de détection comprenant les étapes consistant à :
- mettre en contact un second ligand capable de se lier spécifiquement au brin d'ADN marqué avec le brin d'ADN marqué synthétisé dans des conditions facilitant la liaison spécifique du second ligand au brin d'ADN, le second ligand comprenant une fraction de liaison capable de se lier spécifiquement à l'ADN marqué synthétisé et une enzyme attachée de manière covalente ;
- éliminer le second ligand non lié de l'échantillon ;
- ajouter un substrat pour l'enzyme attachée qui, lors du clivage par l'enzyme attachée, génère un signal détectable ; et
- détecter ledit signal.

8. Procédé selon la revendication 7, dans lequel ledit second ligand est un anticorps qui se lie spécifiquement à l'ADN marqué synthétisé ayant une enzyme, telle que la phosphatase alcaline ou la peroxydase de raifort, attachée de manière covalente à celui-ci.

9. Procédé selon la revendication 8, dans lequel l'enzyme attachée de manière covalente agit sur un substrat pour générer un signal colorimétrique, fluorescent, chimioluminescent ou électrochimique.

10. Procédé selon l'une quelconque des revendications précédentes, dans lequel la détection de l'analyte d'intérêt est une détection quantitative.

11. Kit d'éléments comprenant un premier ligand comprenant une fraction de liaison, capable de se lier spécifiquement à un analyte d'intérêt, et un oligo-désoxyribonucléotide d'amorce d'une longueur de 18 à 40 nucléotides lié à la fraction de liaison ; un polyribonucléotide matrice plus long que l'oligo-désoxyribonucléotide d'amorce et ayant une séquence qui est au moins partiellement complémentaire de l'oligo-désoxyribonucléotide d'amorce, une pluralité de désoxyribonucléotides triphosphates marqués ; et une enzyme ayant une activité de transcriptase inverse.

12. Kit d'éléments selon la revendication 11, dans lequel l'oligo-désoxyribonucléotide d'amorce est choisi parmi les oligo-désoxyribonucléotides contenant les quatre bases, A, G, T et C ou un sous-ensemble de celles-ci ou parmi les oligo-désoxyribonucléotides contenant une seule base, de préférence l'oligo-désoxythymidine.

13. Kit d'éléments selon l'une quelconque des revendications 11 à 12, dans lequel les désoxyribonucléotides triphosphates marqués sont des désoxyuridine triphosphates marqués (dUTP), tels que le bromo-dUTP ou l'iodo-dUTP, de préférence le bromo-dUTP, et le polyribonucléotide matrice est l'acide polyriboadénylique.

14. Kit d'éléments selon l'une quelconque des revendications 11 à 13, dans lequel le polyribonucléotide matrice a une longueur de 10² à 10³ nucléotides, de préférence 2*10² à 5*10² nucléotides.

15. Kit d'éléments selon l'une quelconque des revendications 11 à 14, dans lequel l'enzyme ayant une activité de transcriptase inverse est une polymérase eucaryote, virale ou bactérienne, telle que l'ADN polymérase gamma, la transcriptase inverse du VIH ou du M-MuLV, le fragment de Klenow de l'ADN polymérase I d'*E. coli* ou Bst 3.0 dérivée de *Bacillus Stearothermophilus.*

16. Kit d'éléments selon l'une quelconque des revendications 11 à 15, comprenant en outre un second ligand capable de se lier spécifiquement aux désoxyribonucléotides marqués, ledit second ligand comprenant également une enzyme attachée de manière covalente ; et un substrat pour ladite enzyme.

17. Kit d'éléments selon la revendication 16, dans lequel ledit second ligand est un anticorps monoclonal auquel est attachée de manière covalente une enzyme, telle que la phosphatase alcaline ou la peroxydase de raifort.
